## Europäisches Patentamt
## European Patent Office
## Office européen des brevets

(11) Numéro de publication : **0 316 993 B1**

(12) **FASCICULE DE BREVET EUROPEEN**

(45) Date de publication du fascicule du brevet :
**24.07.91 Bulletin 91/30**

(51) Int. Cl.$^5$ : **A61K 31/22**

(21) Numéro de dépôt : **88202492.0**

(22) Date de dépôt : **08.11.88**

(54) **Compositions pharmaceutiques contenant un dérivé de l'acide 3-hydroxybutanoique choisi parmi les oligomères de cet acide et les esters de cet acide ou de ces oligomères avec du 1,3-butanediol.**

(30) Priorité : **19.11.87 BE 8701314**

(43) Date de publication de la demande :
**24.05.89 Bulletin 89/21**

(45) Mention de la délivrance du brevet :
**24.07.91 Bulletin 91/30**

(84) Etats contractants désignés :
**AT BE CH DE ES FR GB GR IT LI LU NL SE**

(56) Documents cités :
**EP-A- 0 087 192**
**EP-A- 0 249 667**
**DE-A- 2 500 310**
**DE-A- 2 500 312**

(73) Titulaire : **SOLVAY & Cie (Société Anonyme)**
**Rue du Prince Albert, 33**
**B-1050 Bruxelles (BE)**

(72) Inventeur : **Nichels, William**
**Belgielaan, 2A**
**B-1800 Vilvoorde (BE)**
Inventeur : **d'Oultremont, Philippe**
**Château de la Catoire**
**B-7922 Blicquy (BE)**

(74) Mandataire : **Lechien, Monique et al**
**Solvay & Cie S.A. Département de la Propriété**
**Industrielle Rue de Ransbeek, 310**
**B-1120 Bruxelles (BE)**

Jouve, 18, rue Saint-Denis, 75001 PARIS

## Description

La présente invention concerne des compositions pharmaceutiques convenant notamment pour l'épuration sanguine, en particulier des solutions de dialyse mises en oeuvre lors de traitements appliqués chez des personnes atteintes d'insuffisance rénale.

Un certain nombre de maladies des reins conduisent à une insuffisance rénale chronique, la capacité de concentration de l'urine et les possibilités d'excrétion sont altérées ; il en résulte des troubles cardio-vasculaires, digestifs et neuromusculaires. Lorsqu'une transplantation ne peut pas être pratiquée, l'hémodialyse et la dialyse péritonéale permettent de suppléer à la fonction rénale. L'insuffisance rénale aiguë, certains empoisonnements, le coma hépatique, des troubles électrolytiques sévères peuvent également être traités par dialyse.

Les solutions pour dialyse contiennent en général un agent osmotique, des ions, une substance à pouvoir tampon, ainsi que divers additifs éventuels.

L'agent osmotique le plus couramment utilisé est le glucose ; toutefois, une partie de ce glucose peut franchir la membrane péritonéale de sorte que le patient en absorbe 50 à 300 g/jour. Ce phénomène pose de sérieuses difficultés lors de la dialyse de certains patients ; ainsi, il peut perturber le traitement des patients diabétiques et favoriser l'apparition de troubles lipidiques et l'obésité.

Pour remédier à ces inconvénients, on a proposé d'ajouter de l'insuline à la solution de dialyse ou d'employer d'autres agents osmotiques tels que d'autres sucres ou des polyols. Cependant, ces produits présentent également des inconvénients : ils sont toxiques, s'accumulent ou provoquent une polyurie ou une élévation de l'acide lactique. Ainsi, par exemple, le glycérol, qui a été proposé dans la demande de brevet international 82/3987, passe rapidement à travers la membrane péritonéale et nécessite le recours à des concentrations plus élevées que celles du glucose, qui entraînent à terme un gain de poids du patient.

On a maintenant trouvé une composition pharmaceutique utilisable pour l'épuration sanguine qui permet de remédier à ces inconvénients.

La présente invention concerne à cet effet une composition pharmaceutique contenant du dérivé de l'acide 3-hydroxybutanoïque choisi parmi les oligomères de cet acide et les esters de cet acide ou de ces oligomères avec du 1,3-butanediol.

Les oligomères de l'acide 3-hydroxybutanoïque présents dans les compositions selon l'invention peuvent être de n'importe quel type. Généralement, ils sont solubles dans l'eau à température ambiante et à pression atmosphérique. De préférence, on met en oeuvre des oligomères constitués exclusivement ou ayant une teneur élevée en dimères et/ou trimères.

Par esters de l'acide 3-hydroxybutanoïque ou de ces oligomères avec du 1,3-butanediol, on entend tout ester formé à partir de ces composés, quel que soit le type d'isomère mis en oeuvre. Ainsi, l'ester peut être formé à partir de l'un ou l'autre groupement alcool du butanediol, c'est-à-dire le 3-hydroxybutanoate de 3-hydroxy-1-butyle et le 3-hydroxybutanoate de 4-hydroxy-2-butyle, encore appelés 3-mono-(3'-hydroxybutyrate) du 1,3-butanediol et 1-mono-(3'-hydroxybutyrate) du 1,3-butanediol.

L'acide 3-hydroxybutanoïque dont sont dérivés les oligomères et les esters présents dans les compositions selon l'invention, peut être de diverses formes isomériques telles que les formes L(+), D(−) et racémiques. De préférence cependant, il présente la forme isomérique D(−).

L'acide D(−)-3-hydroxybutanoïque et les oligomères de cet acide peuvent être obtenus avantageusement par dépolymérisation de polymères naturels extraits de biomasses selon des procédés tels que notamment décrits dans le brevet européen 0043620.

Les esters de l'acide 3-hydroxybutanoïque ou des oligomères de cet acide avec du 1,3-butanediol peuvent être préparés par toute synthèse organique appropriée.

Ces esters peuvent notamment être préparés par les procédés décrits dans les demandes de brevet allemand DOS 2500310 et DOS 2500312.

Le 1,3-butanediol compris dans ces esters peut être de diverses formes isomériques, telles que les formes L, D et/ou racémiques.

Les compositions selon l'invention peuvent être utilisées comme médicaments et notamment pour le traitement et la prévention en médecine humaine et en médecine vétérinaire d'affections nécessitant une dialyse. Dans ce cas, elles sont formulées pour servir de moyen pharmaceutique destiné à un traitement interne ou externe. Les dérivés de l'acide 3-hydroxybutanoïque selon l'invention sont utilisés pour l'obtention d'un médicament destiné à une utilisation thérapeutique en vue de l'épuration sanguine ou pour la fabrication de moyen destiné à la préparation de solutions pour l'épuration sanguine.

Les compositions peuvent contenir un ou plusieurs dérivés de l'acide 3-hydroxybutanoïque et contiennent habituellement des additifs de formulation permettant de les administrer commodément.

Habituellement, les compositions selon l'invention se présentent sous la forme d'une solution de dialyse. Cette solution aqueuse est à un pH physiologique et peut contenir diverses substances à pouvoir tampon telles que des acides faibles avec leurs bases faibles conjuguées, notamment lactate, acétate, acétoacétate, citrate, bicarbonate, et divers sels physiologiques sous forme d'ions tels que notamment Cl⁻, K⁺, Na⁺, Ca⁺⁺, Mg⁺⁺. Ces sels sont présents dans les solutions de dialyse à des concentrations permettant d'éviter toute déplétion ou surcharge ; c'est-à-dire en concentration suffisante pour être osmotiquement compatible avec le sang.

Les compositions pharmaceutiques selon l'invention peuvent contenir d'autres principes actifs pour l'épuration sanguine, en particulier d'autres agents osmotiques mis en oeuvre dans les solutions pour dialyse. Parmi ceux-ci, on peut citer à titre d'exemples des sucres, des polyols ou d'autres substances telles que des acides aminés, de la gélatine, de l'amidon hydrolysé.

La concentration des dérivés de l'acide 3-hydroxybutanoïque mis en oeuvre dans les compositions selon l'invention est choisie de telle sorte que la force osmotique et l'osmolarité (nombre de particules exprimé en millimoles) soient compatibles avec l'organisme traité. En général, l'osmolarité de la solution de dialyse est comprise entre 250 et 700 milliosmoles par litre, de préférence entre 275 et 600 et de manière particulièrement préférée entre 300 et 500 milliosmoles par litre.

De préférence, la concentration des dérivés de l'acide 3-hydroxybutanoïque mis en oeuvre dans les compositions selon l'invention est comprise entre 0,5 et 200 g par litre de solution de dialyse et de manière particulièrement préférée entre 1 et 100 g/l.

Les dérivés de l'acide 3-hydroxybutanoïque peuvent remplacer le glucose dans toutes les solutions de dialyse, ils constituent des agents osmogènes non toxiques, facilement métabolisables qui n'engendrent pas de désordre métabolique et n'altérent pas la membrane péritonéale. Ils sont particulièrement favorables lors de la dialyse de patients atteints de diabète sucré.

La solution de dialyse selon l'invention convient pour l'hémodialyse, la dialyse péritonéale, la dialyse péritonéale continue ambulatoire, la dialyse péritonéale intermittente.

L'invention est illustrée par les exemples suivants.

## Exemple 1

On prépare une solution pour dialyse péritonéale en mettant en solution dans 1 l d'eau pure, stérile et apyrogène 5,67 g de chlorure de sodium, 3,92 g de lactate de sodium 0,257 g de chlorure de calcium dihydraté, 0,152 g de chlorure de magnésium hexahydraté et 1,32 g de D(−)-3-hydroxybutanoate de 3-hydroxy-1-butyle.

Cette solution est stérilisée par filtration et/ou à la chaleur.

L'osmolarité de cette solution est de 279 milliosmoles/l environ.

## Exemple 2

On prépare une solution pour dialyse péritonéale en mettant en solution dans 1 l d'eau pure, stérile et apyrogène 5,55 g de chlorure de sodium, 1,96 g de lactate de sodium 0,257 g de chlorure de calcium dihydraté, 0,152 g de chlorure de magnésium hexahydraté, 1,42 g de succinate de sodium et 6 g de dimère de l'acide D(−)-3-hydroxybutanoïque.

L'osmolarité de cette solution est de 330 milliosmoles/l environ.

## Exemple 3

On prépare une solution pour dialyse péritonéale destinée à épurer le sang des insuffisants rénaux par soustraction des déchets azotés, du potassium, des phosphates, de l'eau et de volume extracellulaire et à corriger l'acidose par apport de substances tampons.

On mélange dans 1 litre d'eau pure, stérile et apyrogène 5,85 g de chlorure de sodium, 4,75 g d'acétate de sodium 3 $H_2O$, 0,384 g de chlorure de calcium 6 $H_2O$, 0,153 g de chlorure de magnésium 6 $H_2O$ et 14,5 g de D(−)-3-hydroxybutanoate de 4-hydroxy-2-butyle.

L'osmolarité de cette solution est de 360 milliosmoles/l environ.

Cette solution est stérilisée.

## Exemple 4

On prépare une solution pour dialyse péritonéale intermittente en mettant en solution dans 1 litre d'eau pure, stérile et apyrogène 5,85 g de chlorure de sodium, 4,75 g d'acétate de sodium 3 $H_2O$, 0,384 g de chlorure de calcium 6 $H_2O$, 0,153 g de chlorure de magnésium 6 $H_2O$ et 67,5 g de trimère de l'acide D(−)-3-hydroxybutanoïque.

Cette solution est stérilisée.

L'osmolarité de cette solution est de 500 milliosmoles/l environ.

## Revendications

**Revendications pour les Etats Contractants suivants : AT, BE, CH, DE, FR, GB, IT, LI, LU, NL, SE**

1. Compositions pharmaceutiques caractérisées en ce qu'elles contiennent un dérivé de l'acide 3-hydroxybutanoïque choisi parmi les oligomères de cet acide et les esters de cet acide ou de ces oligomères avec du 1,3-butanediol.

2. Compositions pharmaceutiques pour l'épuration sanguine, caractérisées en ce qu'elles contiennent un dérivé de l'acide 3-hydroxybutanoïque choisi parmi les oligomères de cet acide et les esters de cet acide ou de ces oligomères avec du 1,3-butanediol.

3. Compositions selon la revendication 1 ou 2, caractérisées en ce que les oligomères de l'acide 3-hydroxybutanoïque sont constitués exclusivement ou ont une teneur élevée en dimères et/ou en trimères.

4. Compositions selon les revendications 1 à 3, caractérisées en ce que les dérivés proviennent de la forme isomérique D(–) de l'acide 3-hydroxybutanoïque.

5. Compositions selon la revendication 2, 3 ou 4, caractérisées en ce qu'elles comprennent en outre des additifs de formulation et/ou d'autres principes actifs pour l'épuration sanguine.

6. Utilisation d'un dérivé de l'acide 3-hydroxybutanoïque choisi parmi les oligomères de cet acide et les esters de cet acide ou de ces oligomères avec du 1,3-butanediol pour l'obtention d'un médicament destiné à l'épuration sanguine.

7. Utilisation d'un dérivé de l'acide 3-hydroxybutanoïque choisi parmi les oligomères de cet acide et les esters de cet acide ou de ces oligomères avec du 1,3-butanediol pour la fabrication de solution pour l'épuration sanguine.

8. Solution de dialyse convenant pour la dialyse péritonéale comprenant une solution aqueuse de pH physiologique, contenant des sels physiologiques et osmotiquement compatible avec le sang, caractérisée en ce qu'elle contient un dérivé de l'acide 3-hydroxybutanoïque choisi parmi les oligomères de cet acide et les esters de cet acide ou de ces oligomères avec du 1,3-butanediol.

**Revendications pour les Etats Contractants suivants : ES, GR.**

1. Utilisation d'un dérivé de l'acide 3-hydroxybutanoïque choisi parmi les oligomères de cet acide et les esters de cet acide ou de ces oligomères avec du 1,3-butanediol pour l'obtention d'une composition pharmaceutique.

2. Utilisation d'un dérivé de l'acide 3-hydroxybutanoïque choisi parmi les oligomères de cet acide et les esters de cet acide ou de ces oligomères avec du 1,3-butanediol pour l'obtention d'un médicament destiné à l'épuration sanguine.

3. Utilisation d'un dérivé de l'acide 3-hydroxybutanoïque choisi parmi les oligomères de cet acide et les esters de cet acide ou de ces oligomères avec du 1,3-butanediol pour la fabrication de solution pour l'épuration sanguine.

4. Utilisation d'une solution de dialyse convenant pour la dialyse péritonéale comprenant une solution aqueuse de pH physiologique, contenant des sels physiologiques et osmotiquement compatibles avec le sang et un dérivé de l'acide 3-hydroxybutanoïque choisi parmi les oligomères de cet acide et les esters de cet acide ou de ces oligomères avec du 1,3-butanediol.

5. Utilisation selon l'une quelconque des revendications précédentes, caractérisée en ce que l'on utilise les oligomères de l'acide 3-hydroxybutanoïque constitués exclusivement ou ayant une teneur élevée en dimères et/ou en trimères.

6. Utilisation selon l'une quelconque des revendications précédentes, caractérisée en ce que l'on utilise les dérivés provenant de la forme isomérique D(–) de l'acide 3-hydroxybutanoïque.

7. Utilisation selon l'une quelconque des revendications précédentes, caractérisée en ce que l'on utilise en outre des additifs de formulation et/ou d'autres principes actifs pour l'épuration sanguine.

**Patentansprüche**

**Patentansprüche für Vertragsstaaten folgenden : AT, BE, CH, DE, FR, GB, IT, LI, LU, NL, SE**

1. Pharmazeutische Zusammensetzungen dadurch gekennzeichnet, daß sie ein Derivat der 3-Hydroxybuttersäure ausgewählt unter den Oligomeren dieser Säure und den Estern dieser Säure oder dieser Oligomere mit 1,3-Butandiol enthalten.

2. Pharmazeutische Zusammensetzungen zur Blutreinigung, dadurch gekennzeichnet, daß sie ein Derivat der 3-Hydroxybuttersäure ausgewählt unter den Oligomeren dieser Säure und den Estern dieser Säure oder dieser Oligomere mit 1,3-Butandiol enthalten.

3. Zusammensetzungen nach Anspruch 1 oder 2, dadurch gekennzeichnet, daß die Oligomere der 3-Hydroxybuttersäure ausschließlich aus Dimeren und-/oder Trimeren bestehen oder einen erhöhten Gehalt davon aufweisen.

4. Zusammensetzungen nach den Ansprüchen 1 bis 3, dadurch gekennzeichnet, daß die Derivate aus der isomeren Form D(–) der 3-Hydroxybuttersäure stammen.

5. Zusammensetzungen nach Anspruch 2, 3 oder 4, dadurch gekennzeichnet, daß sie außerdem Formulierungszusatzstoffe und/oder andere aktive Bestandteile für die Blutreinigung aufweisen.

6. Verwendung eines Derivats der 3-Hydroxybuttersäure, ausgewählt unter den Oligomeren dieser Säure und den Estern dieser Säure oder dieser Oligomere mit 1,3-Butandiol zum Erhalt eines Medikaments, das zur Blutreinigung bestimmt ist.

7. Verwendung eines Derivats der 3-Hydroxybuttersäure, ausgewählt unter den Oligomeren dieser Säure und den Estern dieser Säure oder dieser Oligomere mit 1,3-Butandiol zur Herstellung einer Lösung für die Blutreinigung.

8. Dialyselösung, die zur peritonealen Dialyse geeignet ist, umfassend eine wässrige Lösung mit physiologischem pH, die mit dem Blut verträgliche physiologische und osmotische Salze enthält, dadurch gekennzeichnet, daß sie ein Derivat der 3-Hydroxybuttersäure, ausgewählt unter den Oligomeren dieser Säure und den Estern dieser Säure oder dieser Oligomere mit 1,3-Butandiol enthält.

## Patentansprüche für Vertragsstaaten folgenden : ES, GR

1. Verwendung eines Derivats der 3-Hydroxybuttersäure, ausgewählt unter den Oligomeren dieser Säure und den Estern dieser Säure oder dieser Oligomere mit 1,3-Butandiol zum Erhalt einer pharmazeutischen Zusammensetzung.

2. Verwendung eines Derivats der 3-Hydroxybuttersäure, ausgewählt unter den Oligomeren dieser Säure und den Estern dieser Säure oder dieser Oligomere mit 1,3-Butandiol zum Erhalt eines Medikamts, das zur Blutreinigung bestimmt ist.

3. Verwendung eines Derivats der 3-Hydroxybuttersäure, ausgewählt unter den Oligomeren dieser Säure und den Estern dieser Säure oder dieser Oligomere mit 1,3-Butandiol zur Herstellung einer Lösung für die Blutreinigung.

4. Verwendung einer Dialyselösung, die zur peritonealen Dialyse geeignet ist, umfassend eine wässrige Lösung mit physiologischem pH, die mit dem Blut verträgliche physiologische und osmotische Salze und ein Derivat der 3-Hydroxybuttersäure, ausgewählt unter den Oligomeren dieser Säure und den Estern dieser Säure oder dieser Oligomere mit 1,3-Butandiol enthält.

5. Verwendung nach einem der vorhergehenden Ansprüche, dadurch gekennzeichnet, daß man die Oligomere der 3-Hydroxybuttersäure, die ausschließlich aus Dimeren und/oder Trimeren bestehen oder einen erhöhten Gehalt davon aufweisen, verwendet.

6. Verwendung nach einem der vorhergehenden Ansprüche, dadurch gekennzeichnet, daß man die Derivate, die aus der isomeren Form D(–) der 3-Hydroxybuttersäure stammen, verwendet.

7. Verwendung nach einem der vorhergehenden Ansprüche, dadurch gekennzeichnet, daß man außerdem Formulierungszusatzstoffe und/oder andere aktive Bestandteile für die Blutreinigung verwendet.

## Claims

### Claims for the following Contracting States : AT, BE, CH, DE, FR, GB, IT, LI, LU, NL, SE

1. Pharmaceutical compositions, characterized in that they contain a derivative of 3-hydroxybutanoic acid chosen from oligomers of this acid and esters of this acid or of these oligomers with 1,3-butanediol.

2. Pharmaceutical compositions for blood purification, characterized in that they contain a derivative of 3-hydroxybutanoic acid chosen from oligomers of this acid and esters of this acid or of these oligomers with 1,3-butanediol.

3. Compositions according to Claim 1 or 2, characterized in that the oligomers of 3-hydroxybutanoic acid consist exclusively or have a high content of dimers and/or of trimers.

4. Compositions according to Claims 1 to 3, characterized in that the derivatives originate from the D(–) isomeric form of 3-hydroxybutanoic acid.

5. Compositions according to Claim 2, 3 or 4, characterized in that they comprise, in addition, formulation additives and/or other active principles for blood purification.

6. Use of a derivative of 3-hydroxybutanoic acid chosen from oligomers of this acid and esters of this acid or of these oligomers with 1,3-butanediol for the production of a medicinal product intended for blood purification.

7. Use of a derivative of 3-hydroxybutanoic acid chosen from oligomers of this acid and esters of this acid or of these oligomers with 1,3-butanediol for the manufacture of solution for blood purification.

8. Dialysis solution suitable for peritoneal dialysis, comprising an aqueous solution of physiological pH containing physiological salts osmotically compatible with the blood, characterized in that it contains a derivative of 3-hydroxybutanoic acid chosen from oligomers of this acid and esters of this acid or of these oligomers with 1,3-butanediol.

### Claims for the following Contracting States : ES, GR

1. Use of a derivative of 3-hydroxybutanoic acid chosen from oligomers of this acid and esters of this acid or of these oligomers with 1,3-butanediol for the production of a pharmaceutical composition.

2. Use of a derivative of 3-hydroxybutanoic acid chosen from oligomers of this acid and esters of this acid or of these oligomers with 1,3-butanediol for the production of a medicinal product intended for blood purification.

3. Use of a derivative of 3-hydroxybutanoic acid chosen from oligomers of this acid and esters of this acid or of these oligomers with 1,3-butanediol for the manufacture of solution for blood purification.

4. Use of a dialysis solution suitable for peritoneal dialysis, comprising an aqueous solution of physiological pH containing physiological salts osmotically compatible with the blood and a derivative of 3-hydroxybutanoic acid chosen from oligomers of this acid and esters of this acid or of these oligomers with 1,3-butanediol.

5. Use according to any one of the preceding claims, characterized in that oligomers of 3-hydroxybutanoic acid consisting exclusively or having a high content of dimers and/or of trimers are used.

6. Use according to any one of the preceding claims, characterized in that derivatives originating from the D(–) isomeric form of 3-hydroxybutanoic acid are used.

7. Use according to any one of the preceding claims, characterized in that, in addition, formulation additives and/or other active principles for blood purification are used.